(19) Europäisches Patentamt
European Patent Office
Office européen des brevets

(11) Veröffentlichungsnummer: **0 061 426**
**B1**

(12) **EUROPÄISCHE PATENTSCHRIFT**

(45) Veröffentlichungstag der Patentschrift: **13.02.85**

(51) Int. Cl.⁴: **C 07 D 207/26**, C 08 K 5/34, C 09 B 57/00, D 06 P 1/642

(21) Anmeldenummer: **82810115.4**

(22) Anmeldetag: **15.03.82**

(54) **Verfahren zum Färben von hochmolekularem organischem Material und neue polycyclische Pigmente.**

(30) Priorität: **20.03.81 CH 1905/81**

(43) Veröffentlichungstag der Anmeldung: **29.09.82 Patentblatt 82/39**

(45) Bekanntmachung des Hinweises auf die Patenterteilung: **13.02.85 Patentblatt 85/7**

(84) Benannte Vertragsstaaten: **BE CH DE FR GB IT LI NL SE**

(56) Entgegenhaltungen:
**TETRAHEDRON LETTERS, Nr. 29, Juli 1974, Seiten 2549-2552, Pergamon Press, G.B., D.G. FARNUM et al.: "Attempted reformatskii reaction of benzonitrile, 1,4-diketo-3,6-diphenylpyrrolo(3,4-C)pyrrole. A lactam analogue of pentalene"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Iqbal, Abul, Dr., Im Schaiengarten 1, CH-4107 Ettingen (CH)**
Erfinder: **Cassar, Luigi, Dr., Violaweg 81, CH-4303 Kaiseraugst (CH)**

**EP 0 061 426 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft das Färben von Polymeren mit Pigmenten. An Pigmente, die für einen breiten Einsatz in Polymeren geeignet sind, werden folgende Anforderungen gestellt: Hohe Reinheit des Farbtons (Sättigung), hohe Farbstärke, hohe Echtheiten, insbesondere Hitzebeständigkeit, und leichte Zugänglichkeit. Die heute gebräuchlichen Rotpigmente genügen zwar einzelnen dieser Anforderungen; eine Kumulierung dieser Vorzüge wird aber von keinem der heutigen Rotpigmente erreicht.

Gegenstand der vorliegenden Erfindung ist nun ein Verfahren zum Färben von hochmolekularem organischem Material in der Masse und ist gekennzeichnet durch die Verwendung eines 1,4-Diketopyrrolo[3,4-c]-pyrrols der Formel

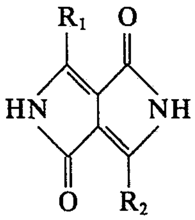

(I)

worin $R_1$ und $R_2$ isocyclische oder heterocyclische aromatische Reste bedeuten. Die Verbindungen der Formel (I) stellen Rotpigmente dar, welche die oben erwähnten Anforderungen in hohem Maße erfüllen.

Formel (I) sowie die nachfolgend aufgeführten Formeln geben nur eine der möglichen tautomeren Strukturen wieder.

Die Reste $R_1$ und $R_2$ können verschieden oder vorzugsweise identisch sein. Bedeuten $R_1$ und $R_2$ aromatische Reste, dann vorzugsweise mono- bis tetracyclische, insbesondere mono- oder bicyclische Reste, also Phenyl-, Diphenylyl- oder Naphthylreste. Diese können die üblichen nicht wasserlöslichmachenden Substituenten aufweisen, wie:

1) Halogenatome, beispielsweise Chlor, Brom oder Fluor.
2) Alkylgruppen, (vorzugsweise mit 1—6 C) diese können nicht wasserlöslichmachende Substituenten aufweisen, wie Fluoratome, Hydroxy-, oder Cyangruppen oder Gruppen der Formeln $-OR_7$, $-OCOR_6$, $-COOR_6$, $-CONR_7R_8$ oder $-R_6-OCONHR_6$, worin $R_6$ Alkyl (vorzugsweise $C_1$—$C_6$-Alkyl), Aryl, beispielsweise Naphthyl oder gegebenenfalls durch Halogen, $C_1$—$C_6$-alkyl oder -alkoxy substituiertes Phenyl, $C_5$—$C_6$-Cycloalkyl, Aralkyl, insbesondere Benzyl oder einen heterocyclischen Rest, $R_7$ und $R_8$ H, Alkyl-, (insbesondere $C_1$—$C_6$-Alkyl), $C_2$—$C_6$-Cyanalkyl und Hydroxyalkyl, $C_5$—$C_6$-Cycloalkyl, Aryl oder Heteroaryl, insbesondere gegebenenfalls durch Halogen, $C_1$—$C_6$-alkyl- oder -alkoxy substituiertes Phenyl, oder worin $R_7$ und $R_8$ zusammen mit dem N-Atom einen 5—6-gliedrigen Heteroring, beispielsweise einen Morpholin- oder Piperidin- oder Phthalimidring bilden. Als weitere Substituenten an den Alkylresten seien ebenfalls mono- oder dialkylierte Aminogruppen, insbesondere mit 2—6 C, Arylreste, beispielsweise Naphthyl- oder insbesondere gegebenenfalls durch Halogen, $C_1$—$C_6$-Alkyl oder -Alkoxy substituierte Phenylreste oder heterocyclische aromatische Reste, wie z. B. die 2-Thienyl-, 2-Benzoxazolyl-, 2-Benzthiazolyl-, 2-Benzimidazolyl, den 6-Benzimidazolonyl-, den 2-, 3- oder 4-Pyridyl-, 2-, 4- oder 6-Chinolylreste.
   Als Beispiele von gegebenenfalls substituierten Alkylresten seien genannt: Methyl, Äthyl, n-Propyl, Isopropyl, Hexyl, Propenyl, Hydroxymethyl, Trifluormethyl, Trifluoräthyl, Cyanmethyl, Methoxycarbonylmethyl, Acetoxymethyl oder Benzyl.
3) Die Gruppe $-OR_9$, worin $R_9$ H, Alkyl, insbesondere $C_1$—$C_6$-Alkyl, Aryl, beispielsweise Naphthyl oder insbesondere gegebenenfalls durch Halogen, $C_1$—$C_6$-Alkyl oder -Alkoxy substituiertes Phenyl, $C_5$—$C_6$-Cycloalkyl, Aralkyl oder einen heterocyclischen Rest bedeutet. Als Beispiele von $R_9$ seien genannt: H, Methyl, Äthyl, n-Propyl, Isopropyl, Trifluoräthyl, Phenyl, o-, m- oder p-Chlorphenyl, o-, m- oder p-Methylphenyl, $\alpha$- oder $\beta$-Naphthyl, Cyclohexyl, Benzyl, Thienyl- oder Pyranylmethyl genannt.
4) Die Gruppe $-SR_9$, worin $R_9$ die unter 3) angegebene Bedeutung hat. Als Beispiele für $R_9$ seien genannt: Methyl, Äthyl, n-Propyl, Isopropyl, Phenyl, o-, m- oder p-Chlorphenyl, o-, m- oder p-Methylphenyl, 2- oder $\beta$-Naphthyl, Cyclohexyl, Benzyl, Thienyl oder Pyranylmethyl.
5) Die Cyangruppe.
6) Die Gruppe der Formel $-NR_7R_8$, worin $R_7$ und $R_8$ die unter 2) angegebene Bedeutung haben. Als Beispiele seien genannt: $NH_2$, Methylamino, Dimethylamino, Äthylamino, Diäthylamino, Isopropylamino, $\beta$-Hydroxyäthylamino, $\beta$-Hydroxypropylamino, N,N-Bis-($\beta$-hydroxyäthyl)-amino, N,N-Bis-($\beta$-cyanäthyl)-amino, Cyclohexylamino, Phenylamino, N-Methylphenylamino, Benzylamino, Dibenzylamino, Piperidyl oder Morpholyl.
7) Die Gruppe der Formel $-COOR_6$, worin $R_6$ die unter 2) angegebene Bedeutung hat. Als Beispiele für $R_6$ seien genannt: Methyl, Äthyl, Isopropyl, n-Butyl, Phenyl, Benzyl oder Furfuryl.
8) Die Gruppe der Formel $-COR_9$, worin $R_9$ die unter 3) angegebene Bedeutung hat. Als Beispiele für $R_9$ seien genannt: H, Methyl, Äthyl, Phenyl, o-, m- oder p-Chlorphenyl, o-, m- oder p-Methylphenyl oder $\alpha$- bzw. $\beta$-Naphthyl.

9) Die Gruppe der Formel $-NR_{10}COR_6$, worin $R_6$ die unter 2) angegebene Bedeutung hat, $R_{10}$ H, Alkyl, insbesondere $C_1-C_6$-Alkyl, Aryl, beispielsweise Naphthyl oder insbesondere gegebenenfalls durch Halogen, $C_1-C_6$-Alkyl oder -Alkoxy substituiertes Phenyl, $C_5-C_6$-Cycloalkyl, Aralkyl oder den Rest $-COR_6$ bedeutet, wobei zwei Reste $-COR_6$ zusammen mit dem N-Atom einen heterocyclischen Ring bilden können. Als Beispiele seien genannt: Acetylamino, Propionylamino, Butyrylamino, Benzoylamino, p-Chlorbenzoylamino, p-Methylbenzoylamino, N-Methyl-acetylamino, N-Methylbenzoylamino, N-Succinimido oder N-Phthalimido.

10) Die Gruppe der Formel $-NR_9COOR_6$, worin $R_6$ und $R_9$ die unter 2) bzw. 3) angegebene Bedeutung haben. Als Beispiele seien die Gruppen $-NHCOOCH_3$, $NHCOOC_2H_5$ oder $NHCOOC_6H_5$ genannt.

11) Die Gruppe der Formel $-NR_9CONR_7R_8$, worin $R_9$, $R_7$ und $R_8$ die unter 3) und 2) angegebene Bedeutung haben. Als Beispiele seien genannt: Ureido, $N_2$-Methylureido, $N_2$-Phenylureido oder $N_2$-2',4'-Dimethylphenylureido.

12) Die Gruppe der Formel $-NHSO_2R_6$, worin $R_6$ die unter 2) angegebene Bedeutung hat. Als Beispiele seien genannt: Methansulfonylamino, Phenylsulfonylamino, p-Toluylsulfonylamino oder $\beta$-Naphthylsulfonylamino.

13) Die Gruppen der Formel $-SO_2R_6$ oder $SOR_6$, worin $R_6$ die unter 2) angegebene Bedeutung hat. Als Beispiele seien genannt: Methylsulfonyl, Äthylsulfonyl, Phenylsulfonyl, 2-Naphthylsulfonyl, Phenylsulfoxidyl.

14) Die Gruppe der Formel $-SO_2OR_{11}$, worin $R_{11}$ einen Arylrest, insbesondere einen gegebenenfalls durch Halogen, $C_1-C_6$-Alkyl oder -Alkoxy substituierten Phenylrest bedeutet. Als Beispiele für $R_{11}$ seien genannt Phenyl, o-, m- oder p-Chlorphenyl, o-, m- oder p-Methylphenyl, $\alpha$- oder $\beta$-Naphthyl.

15) Die Gruppe der Formel $-CONR_7R_8$, worin $R_7$ und $R_8$ die unter 2) angegebene Bedeutung haben. Als Beispiele seien genannt: Carbamoyl, N-Methylcarbamoyl, N-Äthylcarbamoyl, N-Phenylcarbamoyl, N,N-Dimethylcarbamoyl, N-Methyl-N-Phenylcarbamoyl, N-$\alpha$-Naphthylcarbamoyl oder N-Piperidylcarbamoyl.

16) Die Gruppe der Formel $-SO_2NR_7R_8$, worin $R_7$ und $R_8$ die unter 2) angegebene Bedeutung haben. Als Beispiele seien genannt: Sulfamoyl, N-Methylsulfamoyl, N-Äthylsulfamoyl, N-Phenylsulfamoyl, N-Methyl-N-phenylsulfamoyl oder N-Morpholylsulfamoyl.

17) Die Gruppe der Formel $-N{=}N-Q$, worin Q den Rest einer Kupplungskomponente oder einen gegebenenfalls durch Halogen, $C_1-C_6$-Alkyl oder -alkoxy substituierten Phenylrest bedeutet. Als Beispiele für Q seien genannt die Acetoacetarylid-, Pyrazolyl-, Pyridonyl-, o- oder p-Hydroxyphenyl-, o-Hydroxynaphthyl-, p-Aminophenyl-, oder p-N,N-Dimethylaminophenylreste.

18) Die Gruppe der Formel $-OCOR_6$, worin $R_6$ die unter 2) angegebene Bedeutung hat. Als Beispiele für $R_6$ seien genannt Methyl, Äthyl, Phenyl, o-, m- oder p-Chlorphenyl.

19) Die Gruppe der Formel $-OCONHR_6$, worin $R_6$ die unter 2) angegebene Bedeutung hat. Als Beispiele für $R_6$ seien genannt Methyl, Äthyl, Phenyl, o-, m- oder p-Chlorphenyl.

Von besonderem Interesse ist die Verwendung der symmetrischen Verbindungen der Formel (I), worin $R_1$ und $R_2$ einen Rest der Formel

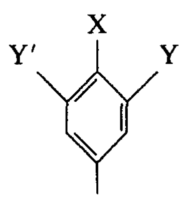

(II)

bedeuten, worin X, Y und Y' H- oder Halogenatome, Carbamoyl-, Cyan-, Trifluormethyl- oder $C_2-C_6$-Alkylcarbamoylgruppen, Alkyl-, Alkoxy- oder Alkylmercaptogruppen mit 1–6 C, Alkoxycarbonyl- oder Alkanoylaminogruppen mit 2–6 C, gegebenenfalls durch Halogen, Alkyl oder Alkoxy mit 1–6 C substituierte Phenoxy-, Phenylmercapto-, Phenoxycarbonyl-, Phenylcarbamoyl- oder Benzoylaminogruppen bedeuten, wobei mindestens einer der Substituenten X, Y und Y' H bedeutet.

Bevorzugt sind symmetrische Verbindungen der Formel (I), worin $R_1$ und $R_2$ Reste der Formel

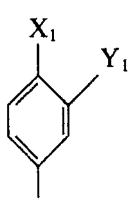

(III)

bedeuten, worin einer der Substituenten $X_1$ und $Y_1$ ein H-, Chlor oder Bromatom, eine Methyl-, Cyan-, oder Alkoxygruppe mit 1–3 C, eine gegebenenfalls durch Chlor oder Methyl substituierte Phenoxygruppe, eine Alkoxycarbonyl- oder Alkylcarbamoylgruppe mit 2–5 C oder eine gegebenenfalls durch Chlor, Methyl oder Methoxy substituierte Phenylcarbamoylgruppe und der andere ein H-Atom bedeutet.

Die Herstellung der Verbindung der Formel (I), worin $R_1$ und $R_2$ Phenyl bedeuten, ist in den Tetrahedron Letters No. 29, S. 2549–52 (1974) durch Erhitzen von Benzonitril mit Bromessigester und Zink in Toluol beschrieben. Die übrigen Verbindungen der Formel (I) sind neu und können ausgehend von substituierten Benzonitrilen auf analoge Weise hergestellt werden, wobei durch nachträgliches Einleiten von Luft die Ausbeute erhöht werden kann.

Die Substituenten in den Resten $R_1$ und $R_2$ der Formel (I) können auch nachträglich eingeführt oder durch Umwandlung anderer Substituenten erhalten werden, beispielsweise durch Halogenierung, Acylierung oder Sulfochlorierung der Verbindung der Formel (I) und nachfolgender Umsetzung des Sulfochlorides mit Aminen, Alkoholen oder Phenolen.

Einen weiteren Erfindungsgegenstand bilden die Verbindungen der Formel

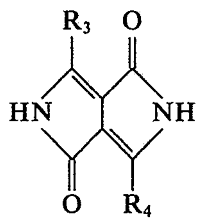

(IV)

worin mindestens einer der Reste $R_3$ und $R_4$ einen substituierten Phenylrest, einen mindestens bicyclischen, carbocyclischen, aromatischen oder einen nicht basischen heterocyclischen, aromatischen Rest bedeutet. Die Reste $R_3$ und $R_4$ können gleich oder verschieden sein.

Bevorzugt sind jene Verbindungen der Formel IV, worin $R_3$ und $R_4$ identisch sind, insbesondere jene worin beide Reste $R_3$ und $R_4$ substituierte Phenylreste bedeuten.

Bevorzugt sind jene symmetrischen Verbindungen der Formel (IV), worin $R_3$ und $R_4$ Reste der Formel

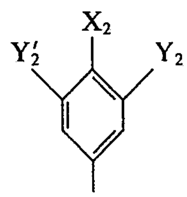

bedeuten, worin $X_2$, $Y_2$ und $Y'_2$ Halogenatome, Hydroxy-, Carbamoyl-, Cyan-, Trifluormethyl- oder $C_1$—$C_6$-Alkylcarbamoylgruppen, Alkyl- oder Alkoxy oder Alkylmercaptogruppen mit 1—6 C, Alkoxycarbonyl- oder Alkanoylaminogruppen mit 2—6 C, gegebenenfalls durch Halogen, Alkyl oder Alkoxy mit 1—6 C substituierte Phenoxy-, Phenylmercapto-, Phenoxycarbonyl-, Phenylcarbamoyl-Benzoylaminogruppen bedeuten, wobei mindestens einer der Substituenten $X_2$, $Y_2$ und $Y'_2$ H bedeutet.

Von besonderem Interesse sind jene symmetrischen Verbindungen der Formel (IV), worin $R_3$ und $R_4$ Reste der Formel

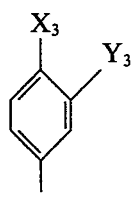

bedeuten, worin einer der Substituenten $X_3$ und $Y_3$ ein Chlor- oder Bromatom, eine Methyl-, Cyano- oder $C_1$—$C_4$-Alkoxygruppe, eine gegebenenfalls durch Chlor oder Methyl substituiertes Phenoxy oder $C_1$—$C_4$-Alkoxycarbonylgruppe und der andere ein H-Atom bedeuten.

Bedeuten $R_3$ und $R_4$ nicht basische Heteroarylreste, dann vorzugsweise mono- bis tricyclische. Diese können rein heterocyclisch sein oder einen heterocyclischen Ring und einen oder mehrere ankondensierte Benzolringe enthalten, welche die oben erwähnten nicht wasserlöslichmachenden Substituenten enthalten können.

Als Beispiele für $R_3$ und $R_4$ in Formel (IV) seien die folgenden Reste genannt:

3- oder 4-Chlorphenyl, 3- oder 4-Bromphenyl, 3- oder 4-Fluorphenyl, 3,4- oder 3,5-Dichlorphenyl, 3- oder 4-Methylphenyl, 3- oder 4-Äthylphenyl, 3- oder 4-Isopropylphenyl, 3,4- oder 3,5-Dimethylphenyl, 3-Chlor-4-methylphenyl, 4-Chlor-3-methylphenyl, 3- oder 4-Methoxyphenyl, 4-Äthoxyphenyl, 3- oder 4-Trifluoräthoxyphenyl, 3,4-Dimethoxyphenyl, 3- oder 4-Äthoxyphenyl, 3-Methyl-4-methoxyphenyl, 4-Methyl-3-methoxyphenyl, 3-Chlor-4-methoxyphenyl, 4-Chlor-3-methoxyphenyl, 3- oder 4-Phenoxyphenyl, 3-Chlor-4-phenoxyphenyl, 3- oder 4-o-Chlorphenoxyphenyl, 3- oder 4-o,m, oder p-Methylphenoxyphenyl,

3- oder 4-Methylmercaptophenyl, 3- oder 4-Phenylmercaptophenyl, 3- oder 4-Cyanphenyl, 3- oder 4-Trifluormethylphenyl, 3,5-Bis-trifluormethylphenyl, 4-Dimethylaminophenyl, 4-Diäthylaminophenyl, 3- oder 4-Methoxycarbonylphenyl, 3- oder 4-Äthoxycarbonylphenyl, 3- oder 4-Phenoxycarbonylphenyl, 3- oder 4-Acetoxyphenyl, 3- oder 4-Benzoylphenyl, 3- oder 4-Benzoyloxyphenyl, 3- oder 4-Acetylaminophenyl, 3- oder 4-Benzoylaminophenyl, 3- oder 4-p-Chlorbenzoylaminophenyl, 4-Phthaloyliminophenyl, 3- oder 4-Carbamoylphenyl, 3- oder 4-N-Methylcarbamoylphenyl, 3- oder 4-N-Äthylcarbamoylphenyl, 3- oder 4-N-Phenylcarbamoylphenyl, 3- oder 4-Ureidophenyl-, 3- oder 4-Sulfamoyl- oder Carbamoylphenyl, 3- oder 4-N-Methylsulfamoylphenyl, 3- oder 4-N-Phenylsulfamoylphenyl, 3- oder 4-Methylsulfonylphenyl, 3- oder 4-Phenylsulfonylphenyl, 4-Ureidophenyl, 4-Methoxycarbonylamino, 4-Alkoxycarbonylamino, 4-Diphenylyl, 2-Naphthyl, 9-Phenanthryl, 3-Pyrenyl, 2-Furoyl, 2-Thienyl, 6-Benzfuranyl, 3,4-Methylendioxyphenyl, 4-Diphenylenoxidyl, 5-Phthalimidyl, $\omega$-Naphthostyrilyl oder 3-Cumarinyl.

Gegenstand der Erfindung ist auch ein Verfahren zur Herstellung der Verbindungen der Formel (IV) durch Erhitzen eines Nitrils der Formel $R_3CN$ gegebenenfalls zusammen mit einem Nitril der Formel $R_4CN$, Bromessigester und Zink in einem inerten organischen Lösungsmittel, vorzugsweise einem aromatischen Kohlenwasserstoff, wie Benzol, Toluol oder Xylol.

Die Reaktion wird vorteilhaft in zwei Stufen durchgeführt. In der ersten Stufe wird zu einer mit Kupfer aktivierten Zinkstaub-Aufschlämmung im Lösungsmittel eine Mischung von Bromessigester, Nitril und Zinkstaub unter Luftausschluß allmählich zugegeben. Dann wird kurze Zeit erwärmt, und dann gegebenenfalls unter Einleiten von Luft längere Zeit erwärmt. Die Verbindung der Formel IV kann durch Abfiltrieren isoliert werden.

Je nach Art ihrer Substituenten und des zu färbenden Polymeren können die Verbindungen der Formel I als polymerlösliche Farbstoffe oder insbesondere als Pigmente verwendet werden. Im letzteren Falle ist es vorteilhaft, die bei der Synthese anfallenden Produkte in eine feindisperse Form überzuführen. Dies kann auf verschiedene Weise geschehen, beispielsweise:

a) durch Mahlen oder Kneten, zweckmäßig in Gegenwart von Mahlhilfsmitteln, wie anorganischen oder organischen Salzen mit oder ohne Zusatz organischer Lösungsmittel. Nach dem Mahlen werden die Hilfsmittel wie üblich entfernt, lösliche anorganische Salze z. B. mit Wasser und wasserunlösliche organische Lösungsmittel beispielsweise durch Wasserdampfdestillation,
b) durch Umfällen aus Schwefelsäure, Methansulfonsäure, Trichloressigsäure oder Polyphosphorsäure.
c) Überführen des Rohpigmentes in ein Alkali- oder Aminsalz und Hydrolyse des letzteren. Dies geschieht beispielsweise durch Anrühren des Rohpigmentes mit einer Base, beispielsweise mit einem Alkalihydroxid, oder -alkoholat, Ammoniak oder einem Amin in einem polaren organischen Lösungsmittel wie Dimethylformamid, wobei das Pigment ganz oder teilweise in Lösung geht. Durch Hydrolyse, vorzugsweise durch Ansäuern der gegebenenfalls filtrierten Lösung wird das Pigment ausgefällt.

Es kann sich als zweckmäßig erweisen, die rohen Pigmente oder die nach a), b) oder c) behandelten Pigmente mit organischen Lösungsmitteln, vorzugsweise mit solchen, die über 100°C sieden, nachzubehandeln.

Als besonders geeignet erweisen sich, durch Halogenatome, Alkyl- oder Nitrogruppen substituierte Benzole, wie Xylole, Chlorbenzol-, o-Dichlorbenzol oder Nitrobenzol sowie Pyridinbasen, wie Pyridin, Picolin oder Chinolin, ferner Ketone, wie Cyclohexanon, Äther wie Äthylenglykolmonomethyl- oder -monoäthyläther, Amide, wie Dimethylformamid oder N-Methyl-pyrrolidon, sowie Dimethylsulfoxyd, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen oder flüssigem Ammoniak oder aliphatischen Aminen durchführen.

Die Pigmente weisen vorzugsweise eine BET-Oberfläche von 5—150 $m^2/g$ auf. Pigmente, die eine BET-Oberfläche von 5—30 $m^2/g$ aufweisen, haben eher deckenden Charakter, während solche mit BET-Oberflächen von 30—150 $m^2/g$ eher transparent sind. Die BET-Oberfläche und die Korngrößenverteilung läßt sich durch die oben erwähnten Nachbehandlungen steuern.

Je nach Verwendungszweck erweist es sich als vorteilhaft, die Pigmente als Toner oder in Form von Präparaten zu verwenden.

Das erfindungsgemäß zu färbende hochmolekulare organische Material kann natürlicher oder künstlicher Herkunft sein. Es kann sich z. B. um Naturharze oder trocknende Öle, Kautschuk oder Casein oder um abgewandelte Naturstoffe, wie Chlorkautschuk, ölmodifizierte Alkydharze, Viscose, um Celluloseäther oder Ester, wie Celluloseacetat, Cellulosepropionat, Celluloseacetobutyrat oder Nitrocellulose handeln, insbesondere aber um vollsynthetische organische Polymere (Duroplaste und Thermoplaste), wie sie durch Polymerisation, Polykondensation oder Polyaddition erhalten werden. Aus der Klasse der

Polymerisationsharze seien in erster Linie genannt, Polyolefine, wie Polyäthylen, Polypropylen, oder Polyisobutylen, ferner substituierte Polyolefine, wie Polymerisate aus Vinylchlorid, Vinylacetat, Styrol, Acrylnitril, der Acrylsäure- und/oder Methacrylsäureester oder Butadien, sowie Kopolymerisate der erwähnten Monomeren, insbesondere ABS oder EVA.

Aus der Reihe der Polyadditionsharze und Polykondensationsharze seien die Kondensationsprodukte von Formaldehyd mit Phenolen, die sogenannten Phenoplaste, und die Kondensationsprodukte von Formaldehyd mit Harnstoff, Thioharnstoff und Melamin, die sogenannten Aminoplaste die als Lackharze verwendeten Polyester, und zwar sowohl gesättigte, wie z. B. Alkydharze, als auch ungesättigte, wie beispielsweise Maleinatharze, ferner die linearen Polyester und Polyamide oder Silikone genannt.

Die erwähnten hochmolekularen Verbindungen können einzeln oder in Gemischen, als plastische Massen oder Schmelzen die gegebenenfalls zu Fasern versponnen werden können, vorliegen.

Sie können auch in Form ihrer Monomeren oder im polymerisierten Zustand in gelöster Form als Filmbildner oder Bindemittel für Lacke oder Druckfarben vorliegen, wie z. B. Leinölfirnis, Nitrocellulose, Alkydharze, Melaminharze und Harnstoff-Formaldehydharze oder Acrylharze.

Die Pigmentierung der hochmolekularen, organischen Substanzen mit den Pigmenten der Formel (I) erfolgt beispielsweise derart, daß man ein solches Pigment gegebenenfalls in Form von Masterbatches, diesen Substraten unter Verwendung von Walzwerken, Misch- oder Mahlapparaten zumischt. Das pigmentierte Material wird hierauf nach an sich bekannten Verfahren wie Kalandrieren, Pressen, Strangpressen, Streichen, Gießen oder durch Spritzguß in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z. B. Ester der Phosphorsäure, Phthalsäure oder Sebacinsäure dienen. Die Weichmacher können im erfindungsgemäßen Verfahren vor oder nach der Einverleibung des Pigmentfarbstoffes in die Polymeren eingearbeitet werden. Es ist ferner möglich, zwecks Erzielung verschiedener Farbtöne den hochmolekularen, organischen Stoffen neben den Verbindungen der Formel (I) noch Füllstoffe bzw. andere farbgebende Bestandteile wie Weiß-, Bunt- oder Schwarzpigmente in beliebigen Mengen zuzufügen.

Zum Pigmentieren von Lacken und Druckfarben werden die hochmolekularen organischen Materialien und die Verbindungen der Formel (I) gegebenenfalls zusammen mit Zusatzstoffen wie Füllmitteln, anderen Pigmenten, Siccativen oder Weichmachern, in einem gemeinsam organischen Lösungsmittel oder Lösungsmittelgemisch fein dispergiert bzw. gelöst. Man kann dabei so verfahren, daß man die einzelnen Komponenten für sich oder auch mehrere gemeinsam dispergiert bzw. löst, und erst hierauf alle Komponente zusammenbringt.

Die erhaltenen Färbungen, beispielsweise in Kunststoffen, Fasern, Lacken oder Drucken zeichnen sich durch einen gelben bis violetten Farbton, eine sehr große Farbstärke, hohe Sättigung, gute Dispergierbarkeit, gute Überlackier-, Migrations-, Hitze-, Licht- und Wetterechtheit sowie durch einen guten Glanz und gutes IR-Remissionsverhalten aus. Besonders interessant sind die erfindungsgemäßen Rot-Pigmente infolge ihres neutralroten Farbtons in Verbindung mit einer hohen Farbstärke, Sättigung und hohen Echtheiten.

Die Verbindungen der Formel (I) können auch als photoelektrophoretische Toner verwendet werden.

Wenn die Verbindungen der Formel (I) in den angewandten Polymeren gelöst vorliegen, zeichnen sie sich ebenfalls durch reinen Farbton, große Farbstärke, hohe Lichtechtheit und außerdem durch hohe Fluoreszenz aus. Sie eignen sich in Sonnenenergie-Kollektoren und zur Herstellung von Laser-Strahlen.

In den nachfolgenden Beispielen bedeuten die Teile, sofern nichts anderes angegeben wird, Gewichtsteile, die Prozente Gewichtsprozente, und die Temperaturen sind in Celsiusgraden angegeben.

## Beispiel 1

(Weich-Polyvinylchlorid)

0,6 g des Pigments der Formel (I) ($R_1$=$R_2$=Phenyl) werden mit 67 g Polyvinylchlorid, 33 g Dioctylphthalat, 2 g Dibutylzinndilaurat und 2 g Titandioxyd zusammengemischt und auf dem Walzenstuhl während 15 Minuten bei 160°C zu einer dünnen Folie verarbeitet. Die so erzeugte rote Färbung ist farbstark, migrations- und lichtecht.

## Beispiel 2

(Hart-Polyvinylchlorid)

0,1 Teil des Pigmentes der Formel (I) ($R_1$=$R_2$=Phenyl) und 1 Teil Titandioxid (Rutil, stabilisiert) werden auf einem Zweiwalzenmischwerk 8 Minuten lang bei einer Walzentemperatur von 160°C und einer Friktion von 1 : 1,2 in 100 Teile Hart-PVC-Mischung bestehend aus 100 Teile PVC-Massepolymerisat, k-Wert 70, 3 Teile Barium-Cadmium-Stearat, 1 Teil tertiäres organisches Phosphit, 1 Teil Octyläthoxystearat und 0,3 Teile Oxystearinsäure eingearbeitet. Das gefärbte Hart-PVC-Fell wird von der Walze abgezogen und 5 Minuten lang bei 160°C auf einer Etagenpresse verpreßt. Die so erhaltene Probeplatte zeichnet sich durch einen sehr leuchtenden, roten Farbton aus.

Beispiel 3

(Polyäthylen)

0,2 Teile des Pigmentes der Formel (I) ($R_1$=$R_2$=Phenyl), 1 Teil Titandioxyd (Rutil) und 100 Teile LD–Polyäthylengranulat werden in einer Trommel gemischt und das Gemisch anschließend auf dem Mischwalzwerk bei 130° verarbeitet. Die Masse wird heiß zu Platten verpreßt oder in der Strangpresse verformt. Die Platten zeigen eine schöne rote Färbung von guter Lichtechtheit.

Beispiel 4

(Polypropylen)

0,1 Teil des Pigments der Formel (I) ($R_1$=$R_2$=Phenyl), 0,5 Teil Titandioxyd (Rutil) und 100 Teile Polypropylengranulat werden in einer Trommel gemischt und das Gemisch anschließend auf dem Mischwalzwert bei 90° verarbeitet bis eine homogen gefärbte Mischung vorliegt. Die Masse wird heiß zu Platten von 1 mm verpreßt. Die Platten zeigen einen schönen roten Farbton von guter Lichtechtheit.

Beispiel 5

(Polystyrol)

0,05 Teile des Pigments der Formel (I) ($R_1$=$R_2$=Phenyl) werden mit 100 Teilen Polystyrol trocken gemischt. Das Gemisch wird bei Temperaturen zwischen 200 und 280°C geknetet, bis eine homogene Einfärbung entstanden ist. Man läßt die gefärbte Masse abkühlen und vermahlt sie in der Mühle zu einer Teilchengröße von etwa 2 bis 4 mm. Das so erhältliche Granulat wird in einer Spritzgußmaschine bei Temperaturen zwischen 220 und 300°C zu Formkörpern verarbeitet. Man erhält rotgefärbte Massen von hoher Lichtechtheit und Temperaturstabilität.

Beispiel 6

(Polymethylmethacrylat)

Mit 100 Teilen Polymethylmethacrylat werden 0,02 Teil des Pigmentes der Formel (I) ($R_1$=$R_2$=p-Chlorphenyl) trocken vermischt und in einem 2-Spindel-Extruder homogenisiert. Das aus dem Mundstück des Extruders austretende Material wird granuliert und kann dann zu Formen auf übliche Weise verpreßt werden. Man erhält einen rotgefärbten Kunststoff.

Beispiel 7

(Polyacrylnitril)

1 Teil des fein verteilten Pigmentes der Formel (I) ($R_1$=$R_2$=Phenyl) wird zu einer Lösung von 165 Teilen Polyacrylnitril in 834 Teile Dimethylformamid gegeben. Die so erhaltene Spinnlösung wird durch eine Düse in ein Fällbad, bestehend aus Wasser von 90° gepreßt. Man erhält eine rot gefärbte Faser von ausgezeichneter Licht-, Wasch- und Chloritechtheit.

Beispiel 8

(Polyamid)

99,5 Teile eines Polyamides aus $\varepsilon$-Caprolactam werden in Form von Schnitzeln mit 0,5 Teil eines feinstverteilten Pigmentes der Formel (I) ($R_1$=$R_2$=Phenyl) trocken paniert. Die so bestäubten Schnitzel werden wie üblich, beispielsweise im Rostspinnverfahren, bei etwa 280 bis 285°C versponnen. Der so erhaltene Faden ist gelb gefärbt, fluoresziert und weist gute Licht- und Naßechtheiten auf.

Beispiel 9

(Polyäthylenterephthalat)

99,9 Teile Polyäthylenterephthalatgranulat wird mit 0,1 Teil des Pigments der Formel (I) ($R_1$=$R_2$=p-Methylphenyl) auf einer Schüttelmaschine während 15 Min. geschüttelt. Das gleichmäßig gefärbte Granulat wird auf einer Schmelzspinnlage bei 285° ±3°C zu Fasern versponnen. Die erhaltenen gelben gefärbten Fasern zeichnen sich durch einen kräftigen reinen Farbton, Fluoreszierung und gute Echtheit aus.

Beispiel 10

(Polycarbonat)

0,05 Teil des Pigmentes der Formel (I) ($R_1$=$R_2$=Phenyl) wird mit 100 Teilen Polycarbonat aus 2,2(4,4'-Dioxydiphenyl)-propan vom K-Wert etwa 50 trocken vermischt und in einem 2-Schnecken-Extruder bei 280°C homogenisiert. Man erhält eine transparente gelbe Färbung von guter Lichtechtheit. Das granulierte Produkt kann nach den üblichen Methoden der thermoplastischen Verformung, so z. B. im Spritzgußverfahren, zu Formteilen verarbeitet werden.

Beispiel 11

(Nitrocellulose)

In einer Stangenmühle werden 40 Teile eines Nitrocelluloselackes, 2,375 Teile Titandioxyd und 0,125 Teile des Pigmentes der Formel (I) ($R_1$=$R_2$=Phenyl) 16 Stunden gemahlen. Der erhaltene Lack wird auf eine Aluminiumfolie in dünner Schicht ausgestrichen. Man erhält einen roten Lackanstrich von sehr guten Echtheiten.

Beispiel 12

(Celluloseacetat)

In einem Kneter behandelt man unter Kühlen eine Mischung aus 25 Teilen des Pigmentes der Formel (I) ($R_1$=$R_2$=Phenyl), 1,25 Teilen Acetylcellulose (54,5% gebundene Essigsäure), 100 Teilen Natriumchlorid und 50 Teilen Diacetonalkohol bis zur Erreichung des gewünschten Feinheitsgrades des Pigmentes. Dann gibt man 25 Teile Wasser hinzu und knetet solange, bis eine feinkörnige Masse entstanden ist. Diese wird auf eine Nutsche gebracht und mit Wasser das Natriumchlorid und der Diacetonalkohol vollständig ausgewaschen. Man trocknet im Vakuumschrank bei 85° und mahlt in einer Hammermühle.

Zu einer aus 100 Teilen Acetylcellulose und 376 Teilen Aceton bestehenden Acetatseidenspinnmasse gibt man 1,33 Teile des erhaltenen Pigmentpräparates. Man verrührt 3 Stunden, was zur vollständigen Verteilung des Farbstoffes genügt. Der nach der üblichen Weise nach dem Trocknungsverfahren aus dieser Masse erhaltene Faden weist eine rote Färbung auf, die eine sehr gute Licht-, Wasch- und Hypochloritechtheit aufweist.

Beispiel 13

(Viskose)

4,8 Teile des Pigments der Formel (I) ($R_1$=$R_2$=Phenyl) werden mit 4,8 Teilen des Natriumsalzes der 1,1'-Dinaphthylmethan-2,2'-disulfonsäure und 22,1 Teilen Wasser solange in einer der bekannten Kolloidmühlen gemahlen, bis alle Farbstoffteilchen kleiner als 1 $\mu$ sind. Die so erhaltene Pigmentsuspension weist einen Pigmentgehalt von ca. 15% auf.

Gibt man diese wässerige Suspension zur Viscose-Spinnmasse, so erhält man nach dem üblichen Spinnprozeß einen rot gefärbten Cellulosefaden, der eine gute Licht- und Hypochloritechtheit aufweist.

Beispiel 14

(Abietinsäure)

In einem Kneter behandelt man unter Kühlen eine Mischung aus 50 Teilen des Pigmentes der Formel (I) ($R_1$=$R_2$=Phenyl), 100 Teilen Staybelite Ester 10 (Glycerinester von hydriertem Kolophonium), 200 Teilen Natriumchlorid und 18 Teilen Diacetonalkohol bis zur Erreichung des gewünschten Feinheitsgrades des Pigmentes. Dann wird durch Einleiten von Wasser von 80° das Natriumchlorid und der Diacetonalkohol aus der Knetmasse entfernt. Die Knetmasse selbst bleibt dabei erhalten. Das salz- und lösungsmittelfreie Knetprodukt wird durch Beheizen des Kneters mit Dampf vollständig getrocknet und nach dem Erkalten im Kneter pulverisiert.

Das Präparat kann beispielsweise zum Anfärben von Lacken eingesetzt werden. Zu diesem Zweck wird das Präparat vorteilhaft mit etwas Toluol angeteigt und die erhaltene Paste zusammen mit dem Lack verrührt.

Beispiel 15

(Harnstoff-Formaldehyd-Harz)

100 Teile eines pulverförmigen Formaldehyd-Harnstoffharzes, das für Preßmassen geeignet ist, 10 Teile Lithopone und 1 Teil des nach Beispiel 1 hergestellten Pigments werden in einer Kugelmühle 16 Stunden gemahlen. Danach wird die Masse bei 140—160° in Formen gepreßt. Die roten Preßlinge besitzen gute Licht- und Hitzebeständigkeit.

Beispiel 16

(Harnstoff-Formaldehyd-Lack)

15 Teile einer 35% Butanol enthaltenden Kolloidwelle, 15 Teile eines mit Ricinusöl modifizierten Phthalatharzes, 15 Teile einer 70%igen butanolischen Lösung eines Harnstofflackharzes, 20 Teile Butylacetat, 10 Teile Glykolmonoäthyläther, 20 Teile Toluol und 5 Teile Alkohol werden zu einem Lack verarbeitet. Dieser wird anschließend mit 2 Teilen des Pigmentes der Formel (I) ($R_1$=$R_2$=Phenyl) und 2 Teile des Titandioxyds (Rutil) pigmentiert und gemahlen. Nach dem Spritzen auf Karton und Trocknen des Lackes erhält man einen roten Überzug von sehr guter Licht-, Überlackier- und Wetterechtheit.

Beispiel 17

(Alkyd-Melamin-Einbrennlack)

60 Teile einer 60%igen Lösung eines nicht-trocknenden Alkydharzes in Xylol (Handelsname Beckosol 27-320 der Firma Reichhold-Albert-Chemie), 36 Teile einer 50%igen Lösung eines Melamin-Formaldehyd-Harzes in einem Butanol-Xylol-Gemisch (Handelsname Super-Beckamin 13-501 der Firma Reichhold-Albert-Chemie), 2 Teile Xylol und 2 Teile Methylcellosolve werden vermischt und 100 Teile dieses Gemisches werden mit Hilfe eines Rührers zu einer homogenen Lacklösung verrührt.

95 Teile des so erhaltenen Klarlackes und 5 Teile des Pigmentes der Formel (I) ($R_1$=$R_2$=Phenyl) werden in einer Kugelmühle während 72 Stunden gemahlen. Der eingefärbte Lack wird dann nach üblicher Spritzmethode auf Blech appliziert und 30 Minuten bei 120°C eingebrannt. Man erhält eine Lackierung von guter Lichtechtheit.

Beispiel 18

(Acryl-Melamin-Einbrennlack)

41,3 Teile einer 60%igen Lösung eines Acrylharzes in Xylol (Handelsname Viacryl VC 373 der Firma Vianova), 16,3 Teile einer 55%igen Lösung eines Melamin-Formaldehyd-Harzes in Butanol (Handelsname Maprenal TTX der Firma Bayer), 32,8 Teile Xylol, 4,6 Teile Äthylglykolacetat, 2 Teile Cyclohexanon, 2 Teile Butylacetat und 1 Teil Siliconöl A (1% in Xylol) der Firma Bayer werden mit Hilfe eines Rührers zu einer homogenen Lacklösung verrührt.

95 Teile des so erhaltenen Klarlackes, 5 Teile des Pigmentes der Formel (I) ($R_1$=$R_2$=Phenyl) werden in einer Kugelmühle während 72 Stunden gemahlen. Der eingefärbte Lack wird dann nach üblicher Spritzmethode auf Blech appliziert und 30 Minuten bei 120°C eingebrannt. Man erhält eine rote Lackierung von guter Lichtechtheit.

## Beispiel 19

(Buchdruck)

1,0 g des Pigments der Formel (I) ($R_1$=$R_2$=p-Chlorphenyl) wird mit 4,0 g Druckfirnis der Zusammensetzung

29,4% Leinöl-Standöl (300 Poise),
67,2% Leinöl-Standöl (20 Poise),
2,1% Kobaltoctoat (8% Co) und
1,3% Bleioctoat (24% Pb)

auf einer Engelsmann-Anreibmaschine fein angerieben und hierauf mit Hilfe eines Klischees im Buchdruckverfahren mit 1 g/$m^2$ auf Kunstdruckpapier gedruckt. Man erhält einen starken Rotton mit guter Transparenz und gutem Glanz.

Das Pigment eignet sich auch für andere Druckverfahren, wie Tiefdruck, Offsetdruck, Flexodruck und ergibt hier ebenfalls sehr gute Resultate.

## Beispiel 20

1 Teil $Cu(OOCCH_3)_2 \cdot H_2O$ wird in einem Erlenmeyerkolben in 25 Vol.-Teilen Eisessig bei 90°C gelöst und anschließend mit 17,97 Teilen Zinkstaub (0,274 Mol) versetzt. Das ganze Gemisch wird 1 Minute lang gut gerührt und anschließend abfiltriert, mit wenig Eisessig und 75 Vol.-Teilen Xylol gewaschen. Das so aktivierte Zink wird sofort in einen Sulfierkolben mit 40 Vol.-Teilen Xylol vorgelegt. Hierauf tropft man unter einer Argon-Schutzgasatmosphäre 15—20 Vol.-Teile einer Mischung von 60 Vol.-Teilen Xylol, 28 Vol.-Teilen Benzonitril (0,27 Mol) und 30,4 Vol.-Teilen Bromessigsäureäthylester (0,27 Mol) zu. Man erwärmt langsam auf 60°C. Es tritt nun eine exotherme Reaktion ein und die Temperatur des Kolbeninhalts steigt rasch auf ca. 90°C. Von jetzt ab hält man die Temperatur bei ~90°C durch Regulierung der Zutropfgeschwindigkeit der restlichen Benzonitril-Bromessigsäureäthylester-Lösung. Nach Beendigung des Zutropfens wird das Reaktionsgemisch ½ Stunde lang bei 90—95°C gerührt. Anschließend entfernt man das Argon und leitet Luft in das Gemisch ein, das am Rückfluß erhitzt und bei derselben Temperatur 10 Stunden lang gerührt wird. Man kühlt dann auf ca. 70°C ab und gibt ca. 100 Vol.-Teile Aceton zu. Das ausgefallene Produkt wird bei Raumtemperatur filtriert, mit Methanol gewaschen und über Nacht bei 80°C unter Vakuum getrocknet. Man erhält 10,16 Teile eines Rohproduktes. 3 Teile dieses Rohproduktes werden in 100 ml Dimethylformamid mit ca. 1,8 Vol.-Teilen 30%iger Natriummethylat-Lösung 2 Stunden bei Raumtemperatur gerührt. Nach Klärfiltration wird das Filtrat mit 3 Vol.-Teilen Eisessig und anschließend mit 13 Vol.-Teilen Wasser versetzt und über Nacht bei Raumtemperatur gerührt. Am nächsten Morgen wird der Niederschlag abfiltriert, mit Dimethylformamid, Äthanol, Wasser und Äthanol gewaschen, und über Nacht unter Vakuum bei 80°C getrocknet. Man erhält 1,5 Teile der Verbindung der Formel I ($R_1$=$R_2$=Phenyl) als rotes Pulver mit einer BET-Oberfläche von 12 $m^2$/g.

Mikroanalyse: $C_{18}H_{12}N_2O_2$ Mol-Gew. 288,3

| | | | |
|---|---|---|---|
| ber. | C 74,99%, | H 4,22%, | N 9,72%, |
| gef. | C 75,0%, | H 4,4%, | N 9,8%. |

UV-spektroskopische Analyse: (Lösungsmittel: N-Methylpyrrolidon)

$\lambda_{max}$ 504 nm; $\varepsilon_{max}$ 33 000.

Das obige Pigment färbt Kunststoffe und Lacke in reinen scharlachroten deckenden Tönen von ausgezeichneter Licht-, Hitze- und Wetterbeständigkeit.

## Beispiel 21

8,8 Teile (0,135 Mol) Zinkstaub werden in eine gut gerührte, 90—95°C heiße Lösung von 0,5 Teil Kupferacetat · $H_2O$ in 12,5 Vol.-Teilen Eisessig gegeben. Nach einer Minute läßt man den entstandenen Schlamm während 1 Minute absetzen und dekantiert den Eisessig so gut wie möglich ab. Der Rückstand wird einmal mit 12,5 Vol.-Teilen Eisessig und anschließend dreimal mit je 25 Vol.-Teilen Xylol gewaschen. Der zurückbleibende graue Schlamm wird zusammen mit 65 ml Xylol und 18,6 Teilen (0,135 Mol) p-Chlorbenzonitril in einen Sulfierkolben vorgelegt. Das Gemisch wird auf ~70°C erwärmt und anschließend 15 Vol.-Teile Bromessigsäureäthylester während ca. 30 Minuten so zugetropft, daß

die Temperatur des Kolbeninhalts 80–85°C nicht übersteigt. Nach beendetem Zutropfen wird am Rückfluß erhitzt und, unter Einleiten von Luft, das Reaktionsgemisch über Nacht bei derselben Temperatur gerührt. Am nächsten Morgen kühlt man auf Raumtemperatur ab und gibt unter Rühren 50 Vol.-Teile Aceton hinzu. Nach ca. 15 Minuten wird abfiltriert und das Nutschgut mit Aceton gewaschen. Das so erhaltene Rohprodukt wird in 250 Vol.-Teilen DMF vorgelegt und mit 5 Vol.-Teilen 30%iger Natriummethylat-Lösung versetzt und 5 Minuten lang gerührt. Nach Klärfiltration wird die alkalische reine Dimethylformamid-Lösung mit 8 Vol.-Teilen Eisessig angesäuert und mit 30 Vol.-Teilen Wasser verdünnt und über Nacht bei Raumtemperatur gerührt. Der rote Niederschlag wird abfiltriert, mit Methanol gewaschen und über Nacht bei 80°C unter Vakuum getrocknet. Man erhält 3,6 Vol.-Teile (15% der Theorie) der Verbindung der Formel I ($R_1$=$R_2$=p-Chlorphenyl) als rotes Pulver.

Elementaranalyse: $C_{18}H_{10}Cl_2N_2O_2$ Mol-Gew. 357,21

| | | | | | |
|---|---|---|---|---|---|
| ber. | C 60,53%, | H 2,82%, | N 7,84%, | O 8,96%, | Cl 19,85%, |
| gef. | C 60,0%, | H 2,8%, | N 7,7%, | O 9,6%, | Cl 19,6%. |

UV-spektroskopische Analyse: (Lösungsmittel N-Methylpyrrolidon)

$\lambda_{max}$ 514 nm; $\varepsilon_{max}$ 33 000.

Die obige Verbindung färbt Kunststoffe und Lacke in sehr reinen, farbstarken blaustichig roten Tönen von ausgezeichneter Licht-, Wetter- und Hitzebeständigkeit.

## Beispiel 22

Verwendet man im Beispiel 21 m-Chlorbenzonitril anstatt p-Chlorbenzonitril, so erhält man bei analoger Versuchsdurchführung 4 g eines Rohpigments das wie folgt behandelt wird:

## Beispiel 23

### Alkalische Umfällung

4 Teile des obigen Rohpigments werden in 135 Vol.-Teilen Dimethylformamid angeschlämmt, mit 2,5 Vol.-Teilen 30%iger Natriummethylat-Lösung versetzt und während 2 Stunden bei Raumtemperatur gerührt. Anschließend wird die Lösung über »Highflow« klärfiltriert und das Filtrat mit 4 Vol.-Teilen Eisessig neutralisiert. Man gibt 18 Teile Wasser hinzu und die resultierende Suspension wird bei Raumtemperatur über Nacht gerührt. Das ausgefallene Pigment wird abfiltriert, zuerst mit wenig Dimethylformamid und anschließend mit Wasser und Äthanol gewaschen und über Nacht unter Vakuum bei 80°C getrocknet. Man erhält 2,4 Teile eines orange-roten Pulvers der folgenden Formel I ($R_1$=$R_2$=m-Chlorphenyl).

Mikroanalyse: $C_{18}H_{10}Cl_2N_2O_2$ Mol-Gew. 357,2

| | | | | |
|---|---|---|---|---|
| ber. | C 60,53%, | H 2,82%, | Cl 19,85%, | N 7,84%, |
| gef. | C 60,00%, | H 2,80%, | Cl 19,90%, | N 7,80%. |

UV-spektroskopische Analyse: Lösungsmittel: N-Methylpyrrolidon

$\lambda_{max}$: 512 nm; $\varepsilon_{max}$: 27 000.

Das obige Pigment aus der alkalischen Umfällung färbt Kunststoffe und Lacke in reinen orangen Tönen von hoher Migrations-, Licht- und Hitzebeständigkeit.

## Beispiel 24

### Saure Umfällung

1,4 Teile des nach Beispiel 23 alkalisch umgefällten Produktes werden in 70 Vol.-Teilen konzentrierter Schwefelsäure angeschlämmt und während ca. 45 Minuten bei Raumtemperatur gerührt. Nach anschließender Klärfiltration über »High-flow« (Glasfritte) wird das Filtrat in ca. 300 Teile Eis gegossen und 15 Minuten bei Raumtemperatur gerührt. Das ausgefallene Pigment wird abfiltriert, mit Wasser und Äthanol gewaschen und über Nacht unter Vakuum bei 80°C getrocknet. Man erhält 1,2 Teile eines

roten Pulvers, das die gleichen mikroanalytischen, massen-, und UV-spektroskopischen Daten aufweist und die gleiche chemische Struktur besitzt, wie das nach der unter Beispiel 23 durch alkalischen Umfällung erhaltene Pigment. Allerdings erhält man bei Einarbeitung des vorliegenden sauer umgefällten Produktes in Kunststoffen und Lacken nunmehr reine scharlachrote (anstatt orange) Färbungen von ebenfalls ausgezeichneten Pigmentechtheiten.

Das Röntgendiffraktogramm des gemäß Beispiel 24 erhaltenen Pigmentes zeigt bei einem Netzebenenabstand von 3,22 Å, eine Linie sehr starker Intensität, bei 14,3, 6,7 und 3,45 Å Linien starker Intensität, bei 4,72, 4,20, 3,51 und 3,35 Å Linien mittlerer Intensität, bei 5,3 und 2,98 Å Linien schwacher Intensität und bei 3,15 Å eine Linie sehr schwacher Intensität.

Das Röntgendiffraktogramm des gemäß Beispiel 23 erhaltenen Pigmentes zeigt bei 14,2 und 3,7 Å Linien sehr starker Intensität, bei 4,72 Å eine Linie starker Intensität, bei 3,52 Å eine Linie mittlerer Intensität und bei 6,9 Å eine Linie schwacher Intensität.

## Beispiel 25

Verfährt man analog Beispiel 24 mit dem nach Beispiel 20 durch alkalische Umfällung erhaltenen Produkt, so erhält man ein sehr farbstarkes, transparentes rotes Pigment mit einer spez. Oberfläche nach BET von 110 $m^2/g$.

## Beispiel 26

Ersetzt man im Beispiel 21 die verwendete Menge an p-Chlorbenzonitril durch ein äquimolares Gemisch von p-Chlorbenzonitril und p-Methylbenzonitril, so erhält man bei analoger Versuchsdurchführung und Aufarbeitung ein rotes Pigmentgemisch, das laut Elementar- sowie massenspektroskopischer Analyse, neben den Pigmenten der Formel I ($R_1$=$R_2$=p-Chlorphenyl), ($R_1$=$R_2$=p-Methylphenyl) noch die Verbindung der Formel (I) ($R_1$=p-Chlorphenyl, $R_2$=p-Toluyl) enthält.

Massenspektroskopie:

MS: $M^+$ : 336.

Das obige Pigmentgemisch färbt Kunststoffe und Lacke in roten Tönen von hoher Sättigung und ausgezeichneten Pigmentechtheiten.

## Beispiel 27–33

Analog Beispiel 20 erhält man weitere Pigmente der Formel

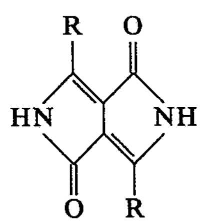

wenn man ein Nitril der Formel

R–CN

mit Bromessigsäureäthylester in Gegenwart von Zn–Cu umsetzt, wobei R die in Kolonne 2 der Tabelle 1 angegebene Bedeutung hat. Kolonne 3 gibt die Nuance der Färbung in Polyvinylchlorid und Kolonne 4 die spektroskopischen Daten der Pigmente wieder.

Tabelle 1

| Beispiel Nr. | R | Nuance in PVC | $\lambda_{max}$*) nm |
|---|---|---|---|
| 27 | 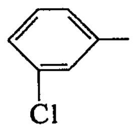 | orange | 512<br>476 |
| 28 | 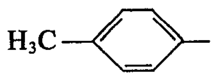 | rot | 507<br>472 |
| 29 | 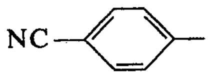 | bordeaux | 535<br>500 |
| 30 | 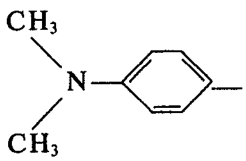 | rot-violett | 554<br>512 |
| 31 | 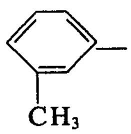 | rot | 505<br>470 |
| 32 | 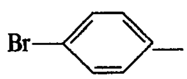 | blaustichig-rot | 515<br>480 |
| 33 | β-Naphthyl | rot | 526<br>491 |
| 34 | 3-Trifluormethylphenyl | Gelb-orange | 474<br>509 |
| 35 | 4-Fluorphenyl | Rot | 464<br>500 |
| 36 | 4-Benzoylaminophenyl | Rot | 474<br>510 |
| 37 | 4-tert.-Butylphenyl | Rot-orange | 472<br>509 |
| 38 | 3,4-Dimethylphenyl | Rot | 472<br>509 |
| 39 | $R_1$ = Phenyl<br>$R_2$ = p-Toluyl | Rot | 470<br>505 |

Fortsetzung

| Beispiel Nr. | R | Nuance in PVC | $\lambda_{max}$*) nm |
|---|---|---|---|
| 40 | 4-Methylsulfonylphenyl | Rot | 525 |
| | | | 488 |
| 41 | 3-Methoxycarbonylphenyl | Rot | 465 |
| | | | 505 |
| 42 | 4-Methoxycarbonylphenyl | Rot-Violett | 495 |
| | | | 520 |
| 43 | 3,4-Dimethoxyphenyl | Rot | 515 |
| | | | 480 |
| 44 | $R_1$=p-Chlorphenyl | Rot | 510 |
| | $R_2$=3,4-Dichlorphenyl | | 480 |
| 45 | 3-Chlor-4-methylphenyl | Rot | 509 |
| | | | 472 |
| 46 | 4-Methoxyphenyl | Rot | |
| 47 | 4-Acetoxyphenyl | Rot | 505 |
| | | | 472 |
| 48 | 4-Acetylaminophenyl | Rot | 525 |
| | | | 485 |
| 49 | 3-Cyanphenyl | Rot | 510 |
| | | | 473 |

*) Alle Messungen wurden in N-Methylpyrrolidon vorgenommen, mit Ausnahme von Beispiel 29, welches in Dimethylformamid durchgeführt wurde.

**Patentansprüche**

1. Verfahren zum Färben von hochmolekularem organischem Material in der Masse, gekennzeichnet durch die Verwendung eines 1,4-Diketopyrrolo[3,4-c]-pyrrols der Formel

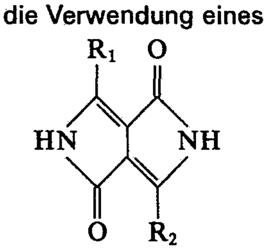

(I)

worin $R_1$ und $R_2$ isocyclische oder heterocyclische aromatische Reste bedeuten.

2. Verfahren gemäß Anspruch 1, dadurch gekennzeichnet, daß man Verbindungen der Formel (I) verwendet, worin $R_1$ und $R_2$ gegebenenfalls substituierte Phenyl- oder Naphthylreste bedeuten.

3. Verfahren gemäß Anspruch 1, dadurch gekennzeichnet, daß man Verbindungen der Formel (I) verwendet, worin $R_1$ und $R_2$ identisch sind.

4. Verfahren gemäß Anspruch 1 und 3, dadurch gekennzeichnet, daß man eine Verbindung der

Formel (I) worin $R_1$ und $R_2$ Reste der Formel

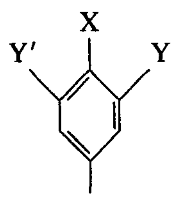

bedeuten, verwendet, worin X, Y und Y' H- oder Halogenatome, Carbamoyl-, Trifluormethyl-, Cyan-oder $C_2$—$C_6$-Alkylcarbamoylgruppen, Alkyl-, Alkoxy-, oder Alkylmercaptogruppen mit 1—6 C, Alkoxycarbonyl- oder Alkanoylamino- oder Dialkylaminogruppen mit 2—6 C, gegebenenfalls durch Halogen, Alkyl oder Alkoxy mit 1—6 C substituierte Phenoxy-, Phenylmercapto-, Phenoxycarbonyl-, Phenylcarbamoyl- oder Benzoylaminogruppen bedeuten und mindestens einer der Substituenten X, Y und Y' ein H-Atom bedeutet.

5. Verfahren gemäß Anspruch 1 und 3, dadurch gekennzeichnet, daß man Verbindungen der Formel (I) verwendet, worin $R_1$ und $R_2$ Reste der Formel

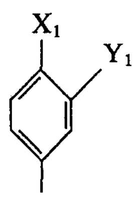

bedeuten, worin einer der Substituenten $X_1$ und $Y_1$ ein H-, Chlor- oder Bromatom, eine Methyl-, Cyan-, oder eine Alkoxygruppe mit 1—3 C, eine gegebenenfalls durch Chlor oder Methyl substituierte Phenoxygruppe, eine Alkoxycarbonyl- oder Alkylcarbamoylgruppe mit 2—5 C oder eine gegebenenfalls durch Chlor, Methyl oder Methoxy substituierte Phenylcarbamoylgruppe und der andere ein H-Atom bedeuten.

6. Verfahren gemäß Anspruch 1, dadurch gekennzeichnet, daß man Verbindungen der Formel (I) mit einer spezifischen Oberfläche nach BET von 5—150 $m^2/g$ verwendet.

7. Verbindungen der Formel

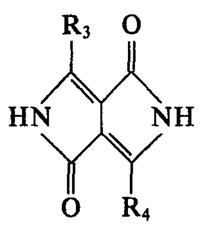

worin mindestens einer der Reste $R_3$ und $R_4$ einen heterocyclischen aromatischen, einen mindestens bicyclischen aromatischen oder einen substituierten Phenylrest bedeutet.

8. Verbindungen gemäß Anspruch 7, worin $R_3$ und $R_4$ substituierte Phenylreste bedeuten.

9. Verbindungen gemäß Anspruch 7, worin $R_3$ und $R_4$ identisch sind.

10. Verbindungen gemäß Ansprüchen 7 und 9, worin $R_3$ und $R_4$ Reste der Formel

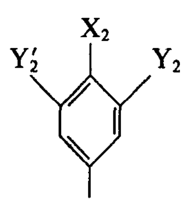

bedeuten, worin $X_2$, $Y_2$ und $Y_2'$ Halogenatome, Hydroxy-, Carbamoyl-, Trifluormethyl oder Cyangruppen, Alkyl-, Alkoxy- oder Alkylmercaptogruppen mit 1—6 C, Alkoxycarbonyl- oder Alkanoylaminogruppen mit 2—6 C, gegebenenfalls durch Halogen, Alkyl oder Alkoxygruppen mit 1—6 C substituierte Phenoxy-, Phenylmercapto-, Phenoxycarbonyl-, Phenylcarbamoyl- oder Benzoylaminogruppen bedeuten und worin mindestens einer der Substituenten $X_2$, $Y_2$ und $Y_2'$ ein H-Atom bedeutet.

11. Verbindungen gemäß Anspruch 7 und 9, worin $R_3$ und $R_4$ Reste der Formel

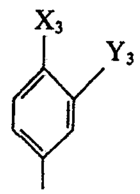

bedeuten, worin einer der Reste $X_3$ und $Y_3$ ein Chlor- oder Bromatom, eine Methyl-, Cyano- oder $C_1-C_4$-Alkoxygruppe, eine gegebenenfalls durch Chlor oder Methyl substituierte Phenoxy- oder eine Alkoxycarbonylgruppe und der andere ein H-Atom bedeuten.

12. Verfahren zur Herstellung von Verbindungen gemäß Anspruch 7, dadurch gekennzeichnet, daß man ein Nitril der Formel $R_3CN$ gegebenenfalls zusammen mit einem Nitril der Formel $R_4CN$, Bromessigester und Zink in einem inerten organischen Lösungsmittel erhitzt.

13. Verfahren gemäß Anspruch 12, dadurch gekennzeichnet, daß man als inertes organisches Lösungsmittel einen aromatischen Kohlenwasserstoff der Benzolreihe verwendet.

14. Verfahren gemäß Anspruch 12, dadurch gekennzeichnet, daß man nach der Zugabe der Reaktionsteilnehmer Luft einleitet.

15. In der Masse gefärbtes hochmolekulares Material enthaltend eine Verbindung der Formel (I).

## Claims

1. A process for dyeing high-molecular organic material in the bulk in which process there is used a 1,4-diketopyrrolo[3,4-c]-pyrrol of the formula

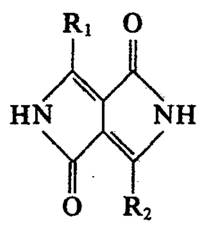

(I)

wherein $R_1$ and $R_2$ are isocyclic or heterocyclic aromatic radicals.

2. A process according to Claim 1, in which there are used compounds of the formula (I), wherein $R_1$ and $R_2$ are unsubstituted or substituted phenyl or naphthyl radicals.

3. A process according to Claim 1, in which there are used compounds of the formula (I), wherein $R_1$ and $R_2$ are identical.

4. A process according to Claims 1 and 3, in which there is used a compound of the formula (I) wherein $R_1$ and $R_2$ are radicals of the formula

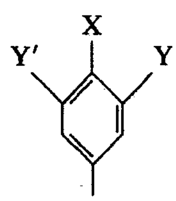

wherein X, Y and Y' are hydrogen or halogen atoms, carbamoyl, trifluoromethyl, cyano or $C_2-C_6$-alkylcarbamoylgroups, alkyl, alkoxy or alkylmercapto groups having 1—6 C atoms, alkoxycarbonyl or alkanoylamino or dialkylamino groups having 2—6 C atoms, phenoxy, phenylmercapto, phenoxycarbonyl, phenylcarbamoyl or benzoylamino groups, each unsubstituted or substituted by halogen, alkyl or alkoxy having 1—6 C atoms, with at least one of the substituents X, Y and Y' being hydrogen.

5. A process according to Claims 1 and 3, in which there is used a compound of the formula (I) wherein $R_1$ and $R_2$ are radicals of the formula

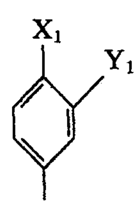

wherein one of the substituents $X_1$ and $Y_1$ is a hydrogen, chlorine or bromine atom, a methyl, cyano or alkoxy group having 1—3 C atoms, a phenoxy group which is unsubstituted or substituted by chlorine or

methyl, or it is an alkoxycarbonyl or alkylcarbamoyl group having 2—5 C atoms, or a phenylcarbamoyl group which is unsubstituted or substituted by chlorine, methyl or methoxy, and the other is a hydrogen atom.

6. A process according to Claim 1, in which there is used a compound of the formula (I) having a specific surface according to BET of 5—150 $m^2/g$.

7. A compound of the formula

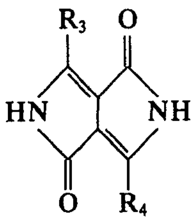

wherein at least one of the radicals $R_3$ and $R_4$ is a heterocyclic aromatic radical, an (at least) bicyclic aromatic radical, or a substituted phenyl radical.

8. A compound according to Claim 7, wherein $R_3$ and $R_4$ are substituted phenyl radicals.

9. A compound according to Claim 7, wherein $R_3$ and $R_4$ are identical.

10. A compound according to Claims 7 and 9, in which $R_3$ and $R_4$ are radicals of the formula

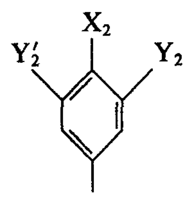

wherein $X_2$, $Y_2$ and $Y_2'$ are halogen atoms, or hydroxyl, carbamoyl, trifluoromethyl or cyano groups, alkyl, alkoxy or alkylmercapto groups having 1—6 C atoms, alkoxycarbonyl or alkanoylamino groups having 2—6 C atoms, phenoxy, phenylmercapto, phenoxycarbonyl, phenylcarbamoyl or benzoylamino groups, each unsubstituted or substituted by halogen, alkyl or alkoxy groups having 1—6 C atoms, and wherein at least one of the substituents $X_2$, $Y_2$ and $Y_2'$ is a hydrogen atom.

11. A compound according to Claims 7 and 9, in which $R_3$ and $R_4$ are radicals of the formula

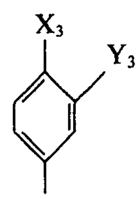

wherein one of the radicals $X_3$ and $Y_3$ is a chlorine or bromine atom, a methyl, cyano or $C_1$—$C_4$-alkoxy group, a phenoxy group unsubstituted or substituted by chlorine or methyl, or it is an alkoxycarbonyl group; and the other is a hydrogen atom.

12. A process for producing a compound according to Claim 7, wherein a nitrile of the formula $R_3CN$, optionally together with a nitrile of the formula $R_4CN$, is heated with bromoacetic ester and zinc in an inert organic solvent.

13. A process according to Claim 12, wherein the inert organic solvent used is an aromatic hydrocarbon of the benzene series.

14. A process according to Claim 12, wherein air is introduced after the addition of the reactants.

15. High-molecular material dyed in the melt, which material contains a compound of the formula (I).

**Revendications**

1. Procédé pour teindre des matières organiques macromoléculaires dans la masse, procédé caractérisé en ce qu'on utilise un dioxo-1,4 pyrrolo[3,4-c]pyrrole répondant à la formule:

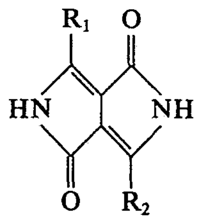

(I)

dans laquelle $R_1$ et $R_2$ représentent chacun un radical aromatique isocyclique ou hétérocyclique.

2. Procédé selon la revendication 1 caractérisé en ce qu'on utilise des composés de formule I dans lesquels $R_1$ et $R_2$ représentent des radicaux phényles ou naphtyles éventuellement substitués.

3. Procédé selon la revendication 1 caractérisé en ce qu'on utilise des composés de formule 1 dans lesquels $R_1$ et $R_2$ sont identiques.

4. Procédé selon l'une des revendications 1 et 3, caractérisé en ce qu'on utilise un composé de formule I dans lequel $R_1$ et $R_2$ représentent des radicaux répondant à la formule suivante:

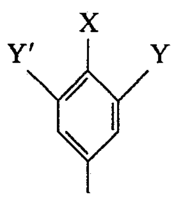

dans laquelle X, Y et Y' représentent chacun un atome d'hydrogène ou d'halogène, un carbamoyle, un trifluorométhyle, un cyano, un alkyl- carbamoyle en $C_2$—$C_6$, un radical alkyle, alcoxy ou alkylthio en $C_1$—$C_6$, un radical alcoxycarbonyle, alcanoylamino ou dialkylamino en $C_2$—$C_6$, ou un radical phénoxy, phénylthio, phénoxycarbonyle, phénylcarbamoyle ou benzoylamino éventuellement porteur d'un halogène, d'un alkyle en $C_1$—$C_6$ ou d'un alcoxy en $C_1$—$C_6$, et au moins l'un des symboles X, Y et Y' représente un atome d'hydrogène.

5. Procédé selon l'une des revendications 1 et 3, caractérisé en ce qu'on utilise des composés de formule I dans lesquels $R_1$ et $R_2$ représentent des radicaux répondant à la formule suivante:

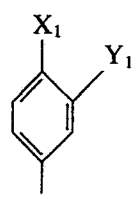

dans laquelle l'un des symboles $X_1$ et $Y_1$ représente un atome d'hydrogène, de chlore ou de brome, un méthyle, un cyano, un alcoxy en $C_1$—$C_3$, un phénoxy éventuellement porteur d'un atome de chlore ou d'un radical méthyle, un radical alcoxycarbonyle ou alkylcarbamoyle contenant de 2 à 5 atomes de carbone, ou un radical phénylcarbamoyle éventuellement porteur d'un atome de chlore ou d'un radical méthyle ou méthoxy, et l'autre représente un atome d'hydrogène.

6. Procédé selon la revendication 1 caractérisé en ce qu'on utilise des composés de formule I qui ont une surface spécifique BET de 5 à 150 $m^2/g$.

7. Composés répondant à la formule:

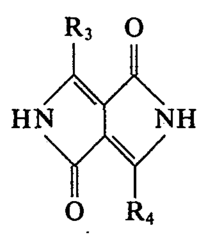

dans laquelle au moins l'un des symboles $R_3$ et $R_4$ représente un radical aromatique hétérocyclique, un radical aromatique au moins bicyclique ou un radical phényle substitué.

8. Composés selon la revendication 7 dans lesquels $R_3$ et $R_4$ représentent des radicaux phényles substitués.

9. Composés selon la revendication 7 dans lesquels $R_3$ et $R_4$ sont identiques.

10. Composés selon l'une des revendications 7 et 9, dans lesquels $R_3$ et $R_4$ représentent des radicaux répondant à la formule:

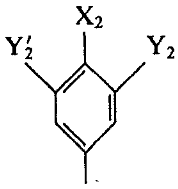

dans laquelle $X_2$, $Y_2$ et $Y'_2$ représentent chacun un halogène, un hydroxy, un carbamoyle, un trifluorométhyle, un cyano, un radical alkyle, alcoxy ou alkylthio contenant de 1 à 6 atomes de carbone, un radical alcoxycarbonyle ou alcanoylamino contenant de 2 à 6 atomes de carbone, ou un radical phénoxy, phénylthio, phénoxycarbonyle, phénylcarbamoyle ou benzoylamino éventuellement porteur d'un halogène, d'un alkyle en $C_1$—$C_6$ ou d'un alcoxy en $C_1$—$C_6$, et dans laquelle au moins l'un des symboles $X_2$, $Y_2$ et $Y'_2$ représente un atome d'hydrogène.

11. Composés selon l'une des revendications 7 et 9, dans lesquels $R_3$ et $R_4$ représentent des radicaux répondant à la formule:

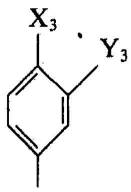

dans laquelle l'un des symboles $X_3$ et $Y_3$ représente un atome de chlore ou de brome, un méthyle, un cyano, un alcoxy en $C_1$—$C_4$, un phénoxy éventuellement porteur d'un atome de chlore ou d'un radical méthyle, ou un radical alcoxycarbonyle et l'autre représente un atome d'hydrogène.

12. Procédé pour préparer des composés selon la revendication 7, caractérisé en ce qu'on chauffe un nitrile de formule $R_3CN$, éventuellement avec un nitrile de formule $R_4CN$, un ester de l'acide bromacétique et du zinc dans un solvant organique inerte.

13. Procédé selon la revendication 12 caractérisé en ce qu'on utilise, comme solvant organique inerte, un hydrocarbure aromatique de la série benzénique.

14. Procédé selon la revendication 12 caractérisé en ce qu'on injecte de l'air après avoir introduit les partenaires réactionnels.

15. Matière macromoléculaire colorée dans la masse, qui contient un composé de formule I.